# EUROPEAN PATENT SPECIFICATION

(11) **EP 3 435 834 B1**
(45) Date of publication and mention of the grant of the patent: **02.06.2021**
(21) Application number: 17776890.0
(22) Date of filing: 01.04.2017
(51) Int. Cl.: A61B 1/00, A61B 1/12, B65D 41/04

(54) **WATER BOTTLE CAP ASSEMBLIES FOR AN ENDOSCOPIC DEVICE**
WASSERFLASCHENKAPPENANORDNUNGEN FÜR EINE ENDOSKOPISCHE VORRICHTUNG
ENSEMBLES BOUCHON DE BOUTEILLE D'EAU POUR UN DISPOSITIF ENDOSCOPIQUE

(30) Priority: 01.04.2016 US 201662317162 P
(43) Date of publication of application: 06.02.2019
(60) Divisional application: 21170696.5
(73) Proprietor: United States Endoscopy Group, Inc., Mentor, OH 44060 (US)
(72) Inventor: MANN, Gary, Mentor OH 44060 (US); KAYE, Christopher, Eastlake OH 44095 (US); MRVA, Joseph, Kirtland OH 44094 (US); HIEBER, Megan, Willoughby OH 44094 (US)
(74) Representative: Petraz, Gilberto Luigi
(86) International application number: PCT/US2017/025655
(87) International publication number: WO 2017/173426

(56) References cited:
- WO-A1-01/85563
- JP-A- 2002 145 312
- US-A- 5 871 111
- US-A- 5 871 111
- US-A1- 2001 044 594
- US-A1- 2003 021 919
- US-A1- 2013 245 377
- US-A1- 2014 316 204
- US-A1- 2014 316 205
- US-A1- 2014 316 205
- US-A1- 2015 080 661
- US-A1- 2015 297 063
- US-B1- 6 485 412
- US-B1- 6 485 412

## Description

### Cross-Reference to Related Applications

This application claims priority to and any benefit of U.S. Patent Application No. 62/317,162, filed April 1, 2016.

### Background

The present disclosure relates to endoscope systems. More particularly, the present disclosure relates to water bottle cap assemblies, wherein the cap assemblies are operative for coupling a water bottle to an endoscope device in order to deliver sterilized water to the endoscope device.

Many invasive medical procedures that previously required major surgery are now performed using endoscopic instruments. Such instruments can provide an internal view of particular body parts, organs, or passages without requiring invasive surgery. Generally, an endoscopic instrument may include one or more channels through which miniaturized, flexible instruments can be inserted and advanced. The endoscope typically includes an elongated flexible insertion tube equipped at one end with an eyepiece or other viewing means and at the other end with an optical lens. The insertion tube transmits images or image-producing signals from the illuminated operative site to the viewing means to provide the instrument operator with full vision of the actions being performed at the instrument's working end.

The insertion tube of an endoscope also provides a flow passage for the delivery of fluid (e.g., liquid or gas) for irrigation, insufflation or other purposes. In conventional practice, it is necessary to provide a flow of sterile water across the optic lens to prevent the buildup of materials (e.g., surgical debris and body fluids) on the optic lens. This flow of water operates, in a sense, like a windshield wiper/washer assembly.

In common designs, an endoscopic instrument typically has a control body which is connected by a light guide tube to a light guide connector, which includes a plurality of connectors that can suitably receive various fittings. For example, the light guide connector can include a connector orifice that receives a grounding lug, a suction port, an air inlet, and a water inlet. As such, the air and water are delivered through the light guide connector, through the light guide tube and into the control body. Alternatively, the control body can also include a water port so as to allow water to be directly provided to the control body. Suitable valves are provided on the control body so as to control the flow of water through the control body and over the optic lens of the instrument. Document US-A-2014/316205 describes a water bottle cap according to the preamble of claim 1. Document US-B-6485412 describes an adapter for the connection of a water bottle to an endoscope. Document US-A-5871111 describes a screwable closure cap with security against over-tightening.

### Summary

Embodiments of the present invention, which is defined in independent claim 1 and the dependent claims 2 - 11, provide water bottle cap assemblies for use in endoscopy procedures. The inventive water bottle cap assemblies can be designed and shaped to function with endoscopic devices generally or may be designed and shaped to function with endoscopic devices having a particular structure unique to a single manufacturer of endoscopic devices. Similarly, the water bottle cap assemblies may be configured for use with a variety of different water sources. In light of their economical nature (and option for disposable, single or daily use), the inventive water bottle cap assemblies allow for provision of a secondary gas in an endoscopy. In one embodiment, the water bottle cap is configured to also support irrigation. These and other benefits of the present invention are more fully described herein.

The present invention provides water bottle cap assemblies that are used with endoscopic devices. In particular, the water bottle cap assemblies allow for in-line placement between the endoscopic device and a water source. In an example not forming part of the claimed invention, the water bottle cap assembly may include a cap comprising a plurality of ports (e.g., two, three, four, etc.) and an engageable member (e.g., internal threads, a sealing ring, an annular sealing platform) configured to sealingly engage with a water source (e.g., a water bottle or suitable container for holding one or more fluids). The assembly also includes a plurality of passage members, each passage member coupled to a respective port so as to be in fluid communication therewith. The tubular members may be single or multiple channels for conveying fluid between the water source and the endoscopic device. In addition, the assembly includes an adaptor coupled to an end of one of the tubular members that is configured to engage with an endoscopic device. At least one of the tubular members may be configured to convey at least one fluid (e.g., water, air, or secondary gas) between the water source and the endoscopic device.

Further features and advantages of the invention will become apparent from the following detailed description made with reference to the accompanying drawings.

### Brief Description of the Drawings

Features and advantages of the general inventive concepts will become apparent from the following detailed description made with reference to the accompanying drawings.
Figure 1 is a perspective view of a water bottle cap assembly of one embodiment of the present subject matter;
Figure 2A is a top/left view of a cap with two ports of one embodiment of the present subject matter;
Figure 2B is a bottom/left view of the cap in Figure 2A;
Figure 2C is a cross-sectional view of the cap in Figure 2A;
Figure 3A is a cross-sectional view of a cap with single port of another embodiment of the present subject matter;
Figure 3B is a bottom/left view of the cap in Figure 3A;
Figure 3C is a cross-sectional view of the cap in Figure 3A;
Figure 4A is a top/left view of a cap with three ports of a third embodiment of the present subject matter;
Figure 4B is a bottom/left view of the cap in Figure 4A;
Figure 4C is a cross-sectional view of the cap in Figure 4A;
Figure 5 is a cross-sectional view of a multi-channel tube of one embodiment of the present subject matter;
Figures 6 is a perspective view of a passage connector of one embodiment of the present subject matter;
Figure 7A is a perspective view of a first adapter of one embodiment of the present subject matter;
Figure 7B is a cross-sectional view of the first adapter in Figure 7A;
Figure 8A is a perspective view of a first adapter of another embodiment of the present subject matter;
Figure 8B is a cross-sectional view of the first adapter in Figure 8A;
Figure 9A is a perspective view of a second adapter of one embodiment of the present subject matter;
Figure 9B is a perspective view of the second adapter in Figure 9A coupling to an air tube; and
Figure 9C is a cross-sectional view of the second adapter in Figure 9A.

### Detailed Description

This Detailed Description merely describes exemplary embodiments in accordance with the general inventive concepts and is not intended to limit the scope of the invention or the claims in any way. Indeed, the invention as described by the claims is broader than and unlimited by the exemplary embodiments set forth herein, and the terms used in the claims have their full ordinary meaning.

The general inventive concepts will now be described with occasional reference to the exemplary embodiments of the invention. This general inventive concept may, however, be embodied in different forms and should not be construed as limited to the embodiments set forth herein. Rather, these embodiments are provided so that this disclosure will be thorough and complete, and will fully convey the scope of the general inventive concepts to those skilled in the art.

Unless otherwise defined, all technical and scientific terms used herein have the same meaning as commonly understood by one of ordinary skill in the art encompassing the general inventive concepts. The terminology set forth in this detailed description is for describing particular embodiments only and is not intended to be limiting of the general inventive concepts. As used in this detailed description and the appended claims, the singular forms "a," "an," and "the" are intended to include the plural forms as well, unless the context clearly indicates otherwise.

Unless otherwise indicated, all numbers, such as for example, numbers expressing measurements or physical characteristics, used in the specification and claims are to be understood as being modified in all instances by the term "about." Accordingly, unless otherwise indicated, the numerical properties set forth in the specification and claims are approximations that may vary depending on the suitable properties sought to be obtained in embodiments of the invention. Notwithstanding that the numerical ranges and parameters setting forth the broad scope of the general inventive concepts are approximations, the numerical values set forth in the specific examples are reported as precisely as possible. Any numerical values, however, inherently contain certain errors necessarily resulting from error found in their respective measurements.

There is a need for a water bottle cap assembly that is easily manufactured and cost effective. There is also a need for a water bottle cap assembly that is configured for use with a variety of endoscopic instruments, procedures (e.g., lens cleaning, secondary gas, and/or irrigation), and water sources. Moreover, there is a need for a water bottle cap assembly that is disposable so as to minimize cross contamination. Last but not least, there is a need for a multi-channel passage for conveying fluids between a water source and an endoscopic device.

Aspects of the present disclosure relate to water bottle cap assemblies configured for coupling a water bottle to an endoscopic device, e.g., a Pentax® endoscope. As discussed below, the water bottle cap assembly comprises a cap configured to engage to a water bottle and a tubing assembly having at least one adaptor configured to engage the endoscopic device.

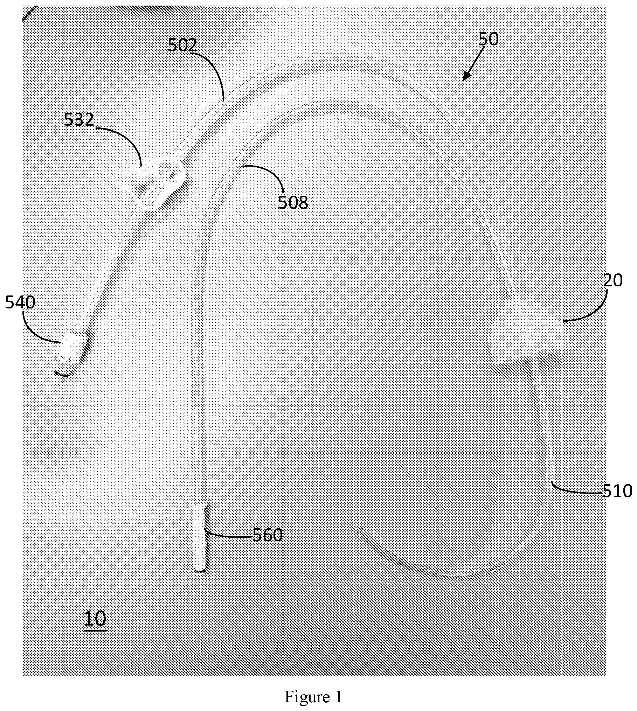

Referring now to the drawings, a water bottle cap assembly 10 is illustrated in Figure 1. The exemplary cap assembly 10 comprises a cap 20 and a passage assembly 50.

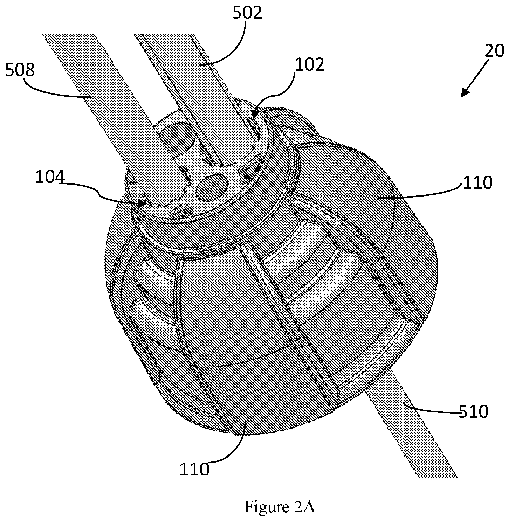

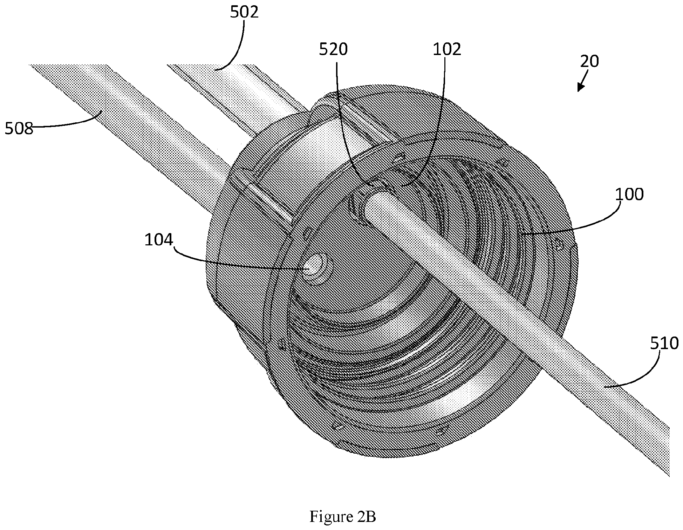

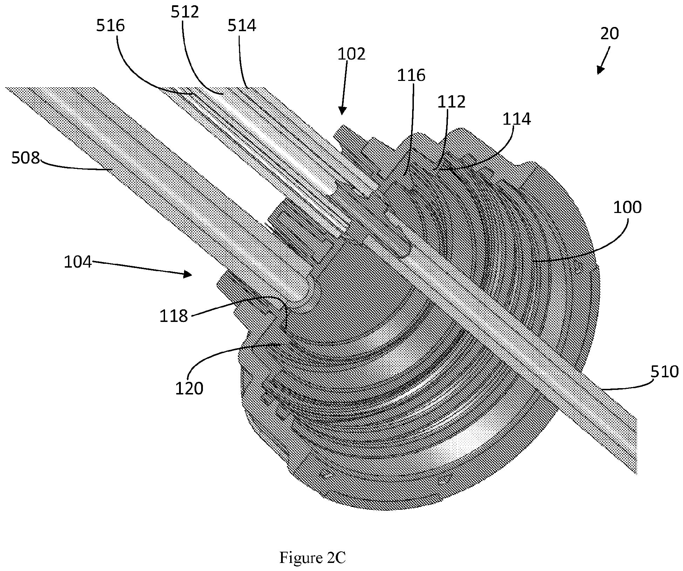

Figures 2A-2C show a cap 20 according to one embodiment of the present application. The cap 20 comprises a first port 102 and a second port 104 both extending outwardly from the exterior surface of the cap 20. The first and second ports 102, 104 extend a sufficient length so as to be configured to engage a passage member for providing or receiving a fluid to an endoscopic device. Each of the ports is configured to engage a respective passage member, wherein each passage member has a single or multiple channels. The passage member is configured to convey fluids between the water bottle and the endoscopic device (e.g., water and air or CO₂). As used herein, the term "fluid" is intended to encompass any material that may be described in relation to flow, such as a gas or a liquid, including solutions or other physical forms of a liquid or a gas that may include some concentration of a solid material in a dissolved, suspended, or otherwise mixed state that does not prevent flow of the liquid or gas.

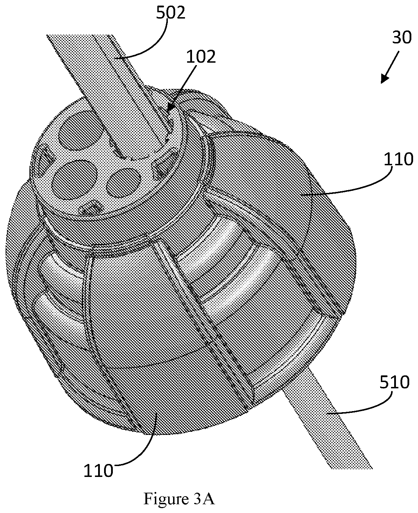

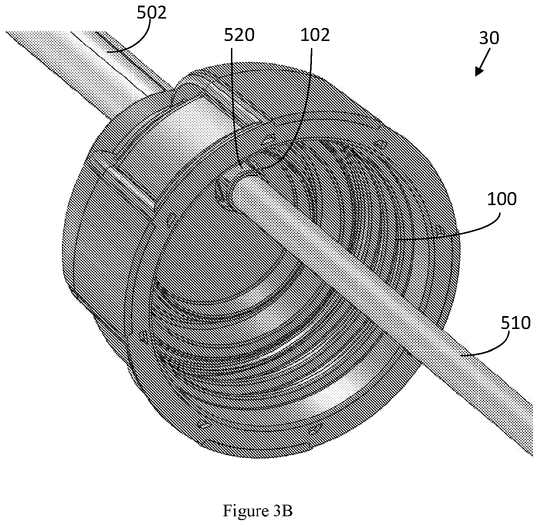

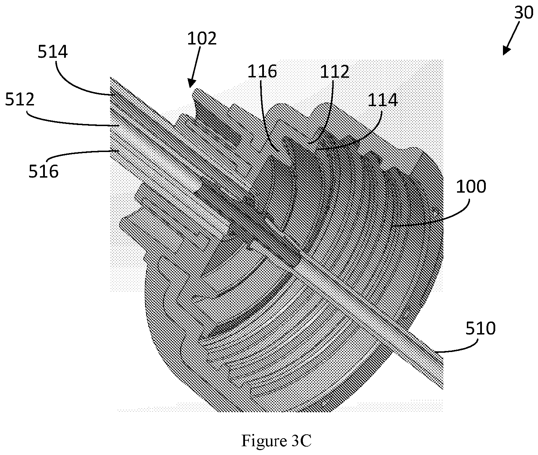

Figures 3A-3C show a cap 30 according to a second embodiment of the present application. The cap 30 comprises a first port 102 extending outwardly from the exterior surface of the cap 30. The first port 102 extends a sufficient length so as to be configured to engage a passage member for providing and/or receiving a fluid to an endoscopic device. The passage member has a single or multiple channels. The passage member is configured to convey fluids between the water bottle and the endoscopic device (e.g., water and air or CO₂).

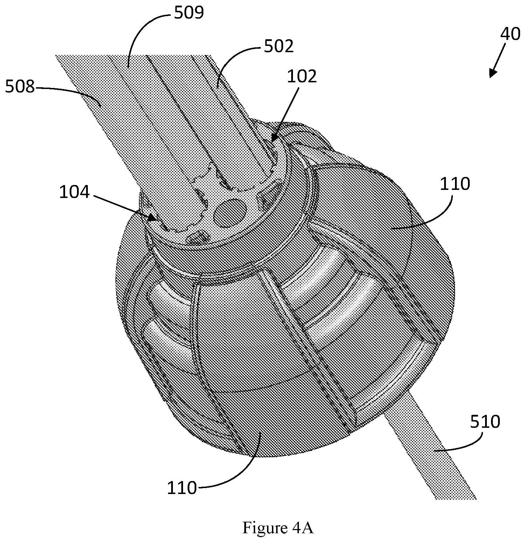

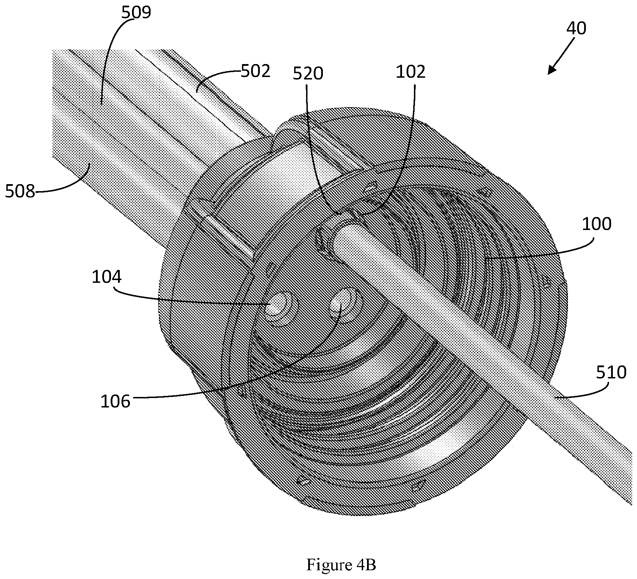

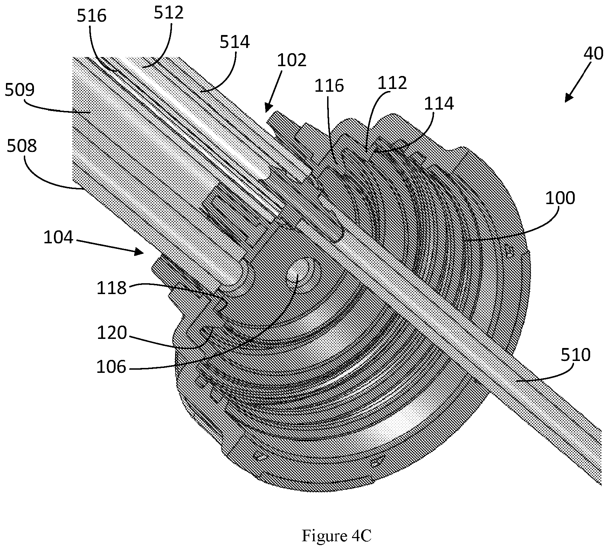

Figures 4A-4C show a cap 40 according to a third embodiment of the present application. The cap 40 comprises a first port 102, a second port 104, and a third port 106 all extending outwardly from the exterior surface of the cap 40. The first, second, and third ports extend a sufficient length so as to be configured to engagement a passage member for providing or receiving a fluid to an endoscopic device. In some embodiments, each of the ports is configured to engage a respective passage member, wherein each passage member has a single or multiple channels. The passage member is configured to convey fluids between the water bottle and the endoscopic device (e.g., water and air or CO₂). In some other embodiments, at least one of the ports is configured to be sealable and may be used to as an access port to inject desired substance into the water bottle.

Referring to Figures 2A-4C, the caps 20, 30, 40 comprise interior threads 100 for engaging external threads on a water bottle. A person skilled in the art may readily understand that the threads could be reversed if desired, i.e., external threads on the cap and internal threads in the water bottle. In this sense, the word "engage", when used in relation to a threaded engagement, is intended to mean a releasable arrangement wherein the various components can be engaged or coupled together by a screwing motion utilizing the threads and also may be detached by unscrewing. In some embodiments, an outer surface of the caps 20, 30, 40 may include a grip 110, such as raised ribs or a knurled surface, for facilitating rotation of the caps 20, 30, 40 by a user. In some embodiments, the caps 20, 30, 40 comprise four grips 110 on the outside surface of the caps 20, 30, 40.

The caps 20, 30, 40 further comprise an annular sealing platform 112. The annular sealing platform 112 extends outwardly from the interior side wall of the caps 20, 30, 40. The annular sealing platform 112 has a certain cross sectional profile. In some embodiments, the annular sealing platform 112 comprises a substantially horizontal contact area 114. The horizontal contact area 114 provides a water-tight seal to a neck of a water bottle when the caps 20, 30, 40 are engaging the water bottle. In some embodiments, the ports 102, 104, 106 are disposed within the annular sealing platform 112.

In some embodiments, the caps 20, 30 40 further comprise a sealing ring 116. The sealing ring 116 extends outwardly from the interior top surface of the caps 20, 30, 40. The sealing ring 116 has a certain cross sectional profile. In some embodiments, the sealing ring 116 is a substantially tapered shape, in which the inner side wall 118 of the sealing ring 116 is substantially vertical and the outer side wall 120 of the sealing ring 116 is substantially inclined. In one embedment, the sealing ring 116 provides a water-tight seal to a neck of a water bottle when the caps 20, 30, 40 are engaging the water bottle. In another embodiment, the outer side wall 120 of the sealing ring 116 and the interior side wall of the cap 20, 30, 40 together provide a water-tight seal to a neck of a water bottle when the caps 20, 30, 40 are engaging the water bottle. In some embodiments, the ports 102, 104, 106 are disposed within the sealing ring 116.

In some embodiments, the sealing ring 116 is closer to the ports 102, 104, 106 than the annular sealing platform 112.

The caps 20, 30, 40 can be made of a plastic material, elastomeric material, and/or any suitable material or combination of materials. The caps 20, 30, 40 comprise a first material and a second material. The second material is softer than the first material. The rigid first material prevents deformation of the cap. The resilient second material facilitates connection of the cap to the water bottle and to maintain a water-tight connection between the cap and the water bottle. Moreover, the resilient second material may be configured to absorb any slack while still maintaining a hermetic seal.

In some embodiments, the grips 110, the annular sealing platform 112, the sealing ring 116, the interior top surface of the caps 20, 30, 40, the interior side wall of the caps 20, 30, 40, the inside and outside surfaces of the first, second, and third ports 102, 104, 106 are made of the second material. In some embodiments, the grips 110, the annular sealing platform 112, the sealing ring 116, the interior top surface of the caps 20, 30, 40, the interior side wall of the caps 20, 30, 40, the inside and outside surfaces of the first, second, and third ports 102, 104, 106 are made in one-piece. In some embodiments, a combination of some of the grips 110, the annular sealing platform 112, the sealing ring 116, the interior top surface of the caps 20, 30, 40, the interior side wall of the caps 20, 30, 40, the inside and outside surfaces of the first, second, and third ports 102, 104, 106 are made in one-piece. In some embodiments, the other parts of the caps 20, 30, 40 are made of the first material. In some embodiments, the second material is overmolded onto the first material and forms the grips 110, the annular sealing platform 112, the sealing ring 116, the interior top surface of the caps 20, 30, 40, the interior side wall of the caps 20, 30, 40, the inside and outside surfaces of the first, second, and third ports 102, 104, 106. In some embodiments, the threads 100 are made of the first material. In some embodiments, the threads 100 are made of the second material. In some other embodiments, the second material and the first material are detachable.

Although the above embodiments only discloses single ports, two ports, and three ports on the caps, a person skilled in the art should understand that a cap may comprise more than three ports. In one embodiment, a first material made base of the cap has four ports. The second material is overmolded over the base and seals the unwanted ports during the manufacturing process. For example, if a cap with two ports is needed, the second material may be overmolded to the base with four ports and seal two of the four ports.

Back to Figures 2A-2C, the passage assembly 50 comprises a multi-channel passage member 502 and a single channel passage member, i.e., an air passage 508.

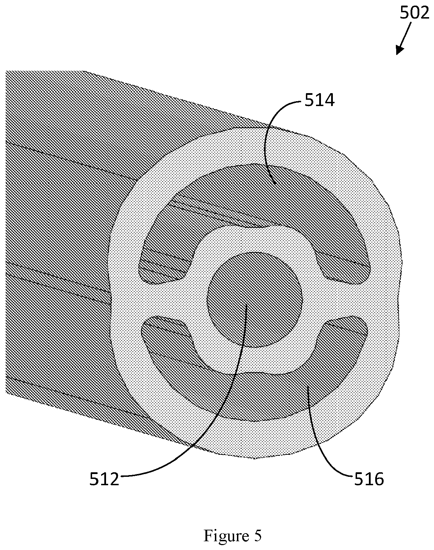

Further referring to the multi-channel passage member 502 in Figure 5, in some embodiments, the multi-channel passage member 502 comprises a inner channel 512 and two C-shape outer channels 514, 516. In some embodiments, the multi-channel passage member 502 comprises a inner channel 512 and three C-shape outer channels. In some embodiments, the multi-channel passage member 502 comprises a inner channel 512 and four C-shape outer channels. In some embodiments, the inner channel 512 and the C-shape outer channels are substantially coaxial. In some embodiments, the outer channels are substantially same shaped or have substantially same cross-sectional area so that the multi-channel passage member 502 has a symmetrical geometry of its cross section. The multi-channel passage member 502 may be made of a variety of materials, including those that are water and CO₂ resistant. In some embodiments, the multi-channel passage member 502 is made in one-piece. A person skilled in the art may understand that the multi-channel passage member 502 may has more than four channels as long as the multi-channel passage member 502 has a symmetrical geometry of its cross section.

Back to Figures 2A-2C, the first port 102 is configured to couple to the multi-channel passage member 502. The second port 104 is configured to receive the air passage 508. The multi-channel passage member 502 is coupled to the first port 102 in a fluid-tight manner, such as using a force-fit connection. In another embodiment, a portion of the outside of the multi-channel passage member 502 is glued to the inside surface of the first port 102. A person skilled in the art may understand that other suitable connection method may be used here. Similarly, the air passage 508 is secured to the second port 104 so as to be in fluid-tight communication.

In some embodiments, the inner channel 512 extends beyond the outer channels 514, 516 and into the water bottle. In some other embodiments, such as the embodiment shown in Figures 2A-C, the inner channel 512 has a similar length as the outer channels 514, 516 and does not extends into the water bottle. A water passage 510 is configured to be disposed inside the cap 20 and is configured to couple to the inner channel 512 via a channel connector 520. As such, the first port 102 is configured to provide air to the endoscopic device through the outer channels 514, 516. The first port 102 is also configured to provide water to the endoscopic device through the inner channel 512. The second port 104 is configured to receive air through the air passage 508 for charging the water within the bottle. Thus, air can be provided into the water bottle to pressurize the water to convey air and/or water to the endoscopic device.

Referring to Figures 3A-3C, the passage assembly 50 comprises a multi-channel passage member 502 shown in Figures 2A-2C. The first port 102 is configured to couple to the multi-channel passage member 502 as shown in Figure 2A-C. In some embodiments, the first port 102 is configured to receive air or CO₂ from a pressurized air or CO₂ source through the outer channels 514, 516 for charging the water within the bottle and to provide water to the endoscopic device through the inner channel 512. Thus, air or CO₂ can be provided into the water bottle to pressurize the water to convey water to the endoscopic device.

Referring to Figures 4A-4C, the passage assembly 50 comprises a multi-channel passage member 502 shown in Figures 2A-2C. The first port 102 is configured to couple to the multi-channel passage member 502 as shown in Figure 2A-C. The second port 104 is configured to couple to the air passage 508 as shown in Figure 2A-C. The third port 106 is configured to couple to a third passage 509. In some embodiments, the third passage 509 has the same configurations as the air passage 508. As such, the first port 102 is configured to provide air to the endoscopic device through the outer channels 514, 516. The first port 102 is also configured to provide water to the endoscopic device through the inner channel 512. The second port 104 is configured to receive air through the air passage 508 for charging the water within the bottle. The third port 106 is configured to provide water from the bottle for, but not limited to, irrigation or cleaning purposes. Thus, air can be provided into the water bottle to pressurize the water to convey air and/or water to the endoscopic device.

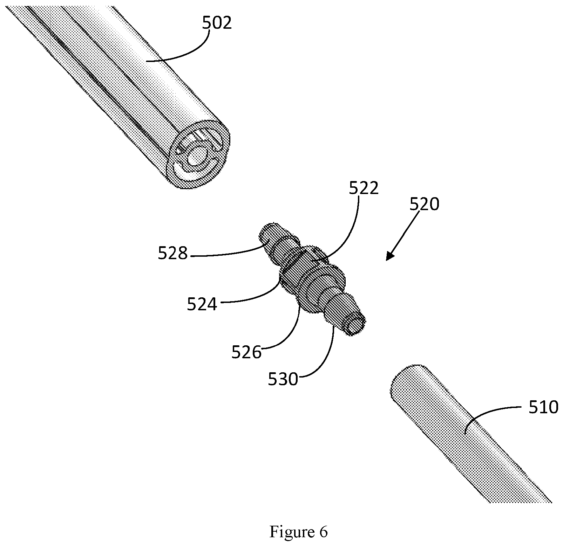

Referring to Figure 6, the channel connector 520 comprises a hollow body 522 with two open ends, two discs 524, 526 disposed at a middle portion of the outer surface of the hollow body 522, and two barbs 528, 530 near each end of the hollow body 522. In some embodiments, the channel connector 520 comprises only one disc 524. The discs 524, 526 ensure both the inner channel 512 and the water passage 510 be able to engage the channel connector 520 at a desired contact length. The discs do not substantially obstruct the outer channels 514, 516. When the water passage 510 is coupled to the inner channel 512 via the channel connector 520, the barbs 528, 530 are used to form the fluid-tight connections.

A pinch clamp 532 may also be provided on the multi-channel passage member 502 that is configured to close off fluid communication between the water bottle and the endoscopic device.

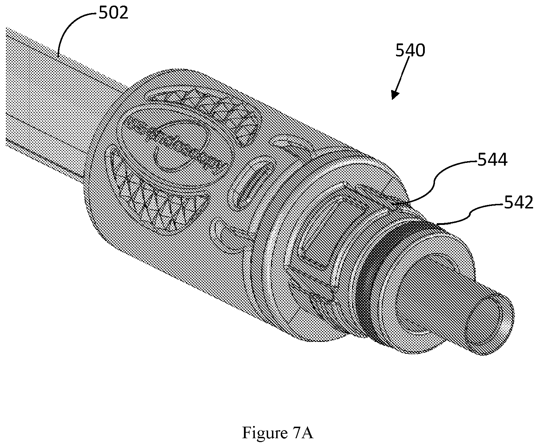

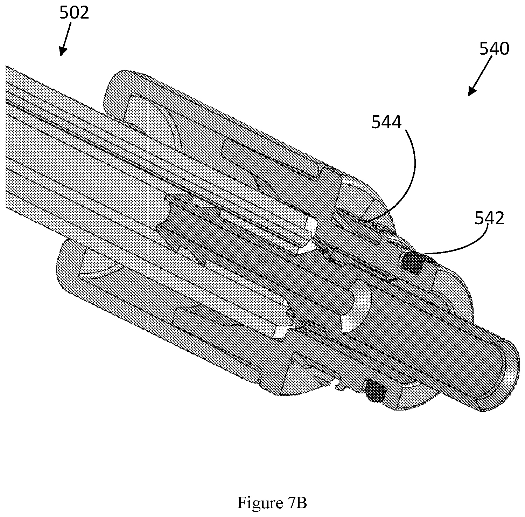

A first embodiment of the first adapter 540 is shown in Figures 7A-7B. The first adapter 540 is configured to couple to the distal end of the multi-channel passage member 502. An o-ring 542 is provided at the distal end of the first adapter 540 for sealing. A molded retention feature 544 is provided in the first adapter 540 and is configured to flex out of the way and snaps back in.

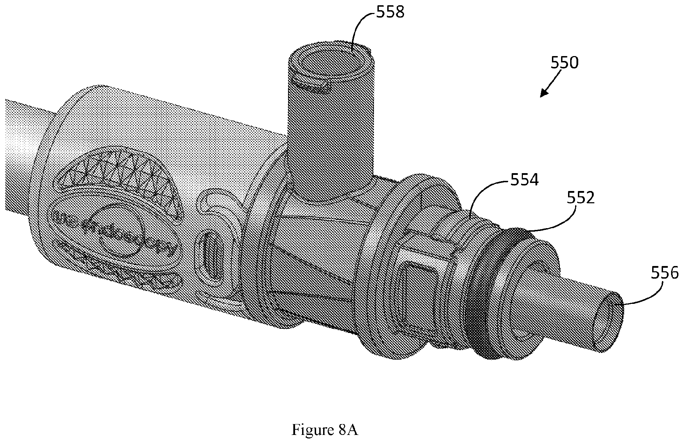

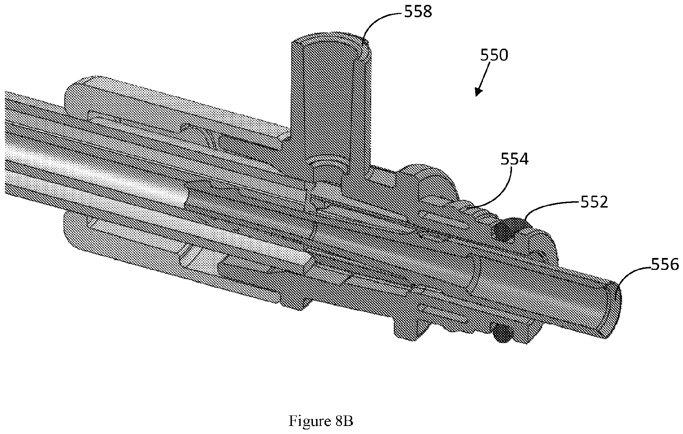

Referring to Figures 8A-8B, another embodiment of the first adapter 550 is shown. The first adapter 550 is configured to form a pathway by which CO₂ can be used for insufflation instead of air. The first adapter 550 has the similar o-ring 552 and molded retention feature 554. The first adapter 550 has a water output 556 and a CO₂ input 558. In one embodiment, the CO₂ input 558 comprises a removable seal cover (not shown). The removable seal cover is configured to seal the CO₂ input 558 when CO₂ is not in use.

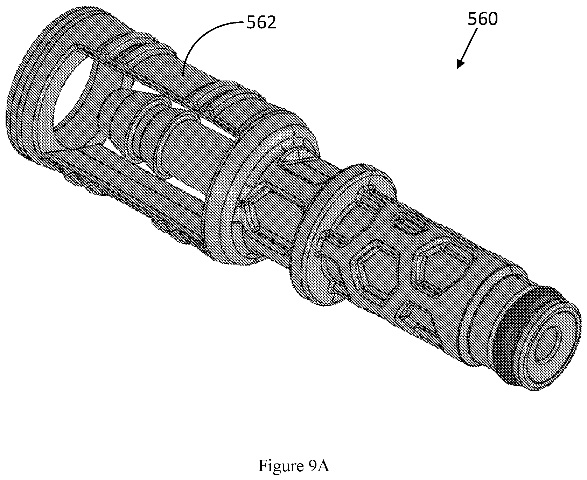

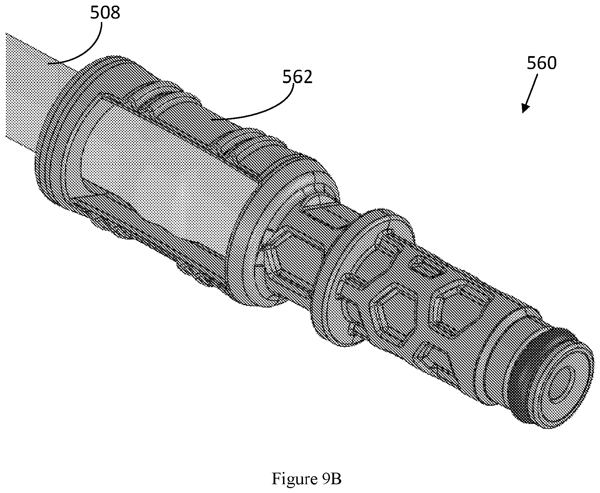

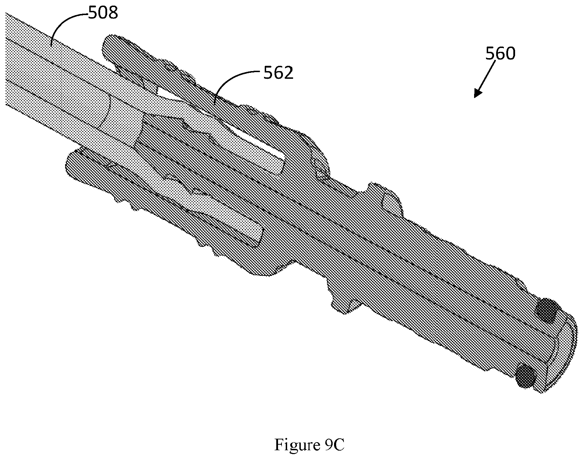

Referring to Figure 9A-9B, a second adapter 560 is shown. The second adapter 560 is configured to couple to the distal end of the air passage 508 and is configured to couple to the Pentax video processor to allow air to flow into the water bottle. The second adapter 560 comprises a cage grip 562. The cage grip 562 at least partially covers the connection portion between the second adapter and the air passage 508. The cage grip 562 helps a user to pull off the second adapter 560 from the video processor without directly pulling the air passage 508.

The present subject matter discloses that a water bottle cap (20) comprises: at least one port, extending outwardly from a exterior surface of the cap, wherein the port extends a predetermined length and is configured to engage a passage member for providing or receiving a fluid to an endoscopic device, interior threads, configured to engage external threads on a water bottle, and an annular sealing platform (112), extending outwardly from a interior side wall of the cap, wherein the annular sealing platform comprises a substantial horizontal contact area (114), configured to provide a water-tight seal to a neck of a water bottle when the cap is engaging the water bottle. The port is disposed within the annular sealing platform (112). The water bottle cap further comprises a sealing ring (116), extending outwardly from an interior top surface of the cap and providing a water-tight seal to a neck of a water bottle when the cap is engaging the water bottle. The sealing ring (116) is a substantially tapered shape. An inner side wall (118) of the sealing ring is substantially vertical, and an outer side wall (120) of the sealing ring is substantially inclined. The outer side wall of the sealing ring and an interior side wall of the cap are configured to provide a water-tight seal to a neck a water bottle when the cap is engaging the water bottle. The port is disposed with the sealing ring (116). The sealing ring (116) is closer to the port than the annular sealing platform (112). The water bottle cap further comprises a grip (110), disposed on an outer surface of the cap. The cap is made of a first material and a second material, wherein the second material is softer than the first material. At least the annular sealing platform (112), the interior top surface of the cap, an interior side wall of the cap, and inside and outside surfaces of the port are made of a second material softer than other parts of the cap made of a first material. At least the annular sealing platform (112), the sealing ring (116), the interior top surface of the cap, the interior side wall of the cap, and inside and outside surfaces of the port are made of a second material softer than other parts of the cap made of a first material. At least the grip (110), the annular sealing platform (112), the interior top surface of the cap, an interior side wall of the cap, and inside and outside surfaces of the port are made of a second material softer than other parts of the cap made of a first material. At least the grip (110), the annular sealing platform (112), the sealing ring (116), the interior top surface of the cap, the interior side wall of the cap, and inside and outside surfaces of the port are made of a second material softer than other parts of the cap made of a first material. The second material is made in one-piece. The second material is overmolded onto the first material.

The present subject matter discloses that a water bottle cap assembly comprises a water bottle cap (20), comprising at least one port, extending outwardly from a exterior surface of the cap, and a passage assembly (50), comprising a multi-channel passage member (502), wherein the port extends a predetermined length and is configured to engage the passage assembly in a fluid-tight manner for providing or receiving a fluid to an endoscopic device. The multi-channel passage member (502) comprises an inner channel (512) and at least one C-shape outer channel. The multi-channel passage member comprises at least two C-shape outer channels (514, 516). Each outer channel has a substantially same cross-sectional area. The multi-channel passage member (502) has a symmetrical geometry of its cross section. The inner channel and the outer channel are coaxial. The multi-channel passage member (502) is made in one-piece. The multi-channel passage member (502) is made of water and CO₂ resistant material. The inner channel (512) extends beyond the outer channels (514, 516) and passes through the cap. The water bottle cap assembly further comprising: a second passage (510), and a channel connector (520), wherein the channel connector (520) comprises: a hollow body (522) with two open ends, two barbs (528, 530), disposed near each open end of the hollow body and configured to form the fluid-tight connections when the second passage is coupled to the inner channel via the channel connector, wherein the inner channel (512) has a similar length as the outer channels (514, 516). The channel connector (520) further comprises at least one disc (524, 526), disposed at a middle portion of the outer surface of the hollow body, wherein the disc is configured to allow the inner channel (512) and the water passage (510) to engage the channel connector at a predetermined contact length.

The present subject matter discloses that an adapter (540, 550) for coupling a multi-channel passage member to an endoscopic device comprises: a first connection portion , configured to couple with the multi-channel passage, a second connection portion, configured to couple with the endoscopic device, wherein the first and second connection portions have at least one fluid communication, an o-ring (542, 552), disposed on the second connection portion and configured to provide sealing between the adapter and the endoscopic device, and a molded retention (544, 554), disposed on the second connection portion and configured to flex out of the way and snaps back in to the endoscopic device. The adapter (550) further comprising a CO₂ input port (558), disposed on the second connection portion, wherein the CO2 input port has a fluid communication with one channel of the multi-channel passage, when the adapter is coupled with the multi-channel passage.

The present subject matter discloses that an adapter (560) for coupling an air passage to a video processor, comprises: a first connection portion , configured to couple with the air passage, a second connection portion, configured to couple with the video processor, and a cage grip (562), partially covering the first connection portion, wherein the cage grip (562) is configured to prevent a user from directly pulling the air passage.

While various inventive aspects, concepts and features of the general inventive concepts are described and illustrated herein in the context of various exemplary embodiments, these various aspects, concepts and features may be used in many alternative embodiments, either individually or in various combinations and subcombinations thereof. Unless expressly excluded herein all such combinations and subcombinations are intended to be within the scope of the general inventive concepts. Still further, while various alternative embodiments as to the various aspects, concepts and features of the inventions (such as alternative materials, structures, configurations, methods, circuits, devices and components, alternatives as to form, fit and function, and so on) may be described herein, such descriptions are not intended to be a complete or exhaustive list of available alternative embodiments, whether presently known or later developed. Those skilled in the art may readily adopt one or more of the inventive aspects, concepts or features into additional embodiments and uses within the scope of the general inventive concepts even if such embodiments are not expressly disclosed herein. Additionally, even though some features, concepts or aspects of the inventions may be described herein as being a preferred arrangement or method, such description is not intended to suggest that such feature is required or necessary unless expressly so stated. Still further, exemplary or representative values and ranges may be included to assist in understanding the present disclosure; however, such values and ranges are not to be construed in a limiting sense and are intended to be critical values or ranges only if so expressly stated. Moreover, while various aspects, features and concepts may be expressly identified herein as being inventive or forming part of an invention, such identification is not intended to be exclusive, but rather there may be inventive aspects, concepts and features that are fully described herein without being expressly identified as such or as part of a specific invention. Descriptions of exemplary methods or processes are not limited to inclusion of all steps as being required in all cases, nor is the order that the steps are presented to be construed as required or necessary unless expressly so stated.

## Claims

1. A water bottle cap (20), comprising:
at least one port, extending outwardly from a exterior surface of the cap, wherein the port extends a predetermined length and is configured to engage a passage member for providing or receiving a fluid to an endoscopic device,
interior threads, configured to engage external threads on a water bottle, and
an annular sealing platform (112), extending outwardly from a interior side wall of the cap, wherein the annular sealing platform comprises a substantial horizontal contact area (114), configured to provide a water-tight seal to a neck of a water bottle when the cap is engaging the water bottle, **characterized in that** at least the annular sealing platform (112), the interior top surface of the cap, an interior side wall of the cap, and inside and outside surfaces of the port are made of a second material softer than other parts of the cap made of a first material.

2. The water bottle cap of claim 1, wherein the port is disposed within the annular sealing platform (112).

3. The water bottle cap of claim 1 further comprising a sealing ring (116), extending outwardly from an interior top surface of the cap and providing a water-tight seal to a neck of a water bottle when the cap is engaging the water bottle.

4. The water bottle cap of claim 3, wherein the sealing ring (116) is a substantially tapered shape.

5. The water bottle cap of claim 3, wherein an inner side wall (118) of the sealing ring is substantially vertical, and an outer side wall (120) of the sealing ring is substantially inclined.

6. The water bottle cap of claim 3, wherein the outer side wall of the sealing ring and an interior side wall of the cap are configured to provide a water-tight seal to a neck a water bottle when the cap is engaging the water bottle.

7. The water bottle cap of claim 3, wherein the sealing ring (116) is closer to the port than the annular sealing platform (112).

8. The water bottle cap of any of claims 1 or 3 further comprising a grip (110), disposed on an outer surface of the cap.

9. The water bottle cap of claim 3, wherein the sealing ring (116) is made of the second material.

10. The water bottle cap of any of the preceding claims 1 to 9, wherein the second material is made in one-piece.

11. The water bottle cap of any of the preceding claims 1 to 10, wherein the second material is overmolded onto the first material.

## Patentansprüche

1. Wasserbehälterverschluss (20)
mit wenigstens einem Anschluss, der sich von einer außenseitigen Oberfläche des Verschlusses nach außen erstreckt, wobei sich der Anschluss über eine vorbestimmte Länge erstreckt und dazu eingerichtet ist, mit einem Durchlassteil in Eingriff zu kommen, um einer endoskopischen Vorrichtung ein Fluid bereitzustellen oder von dieser aufzunehmen,
mit innenseitigen Gewindegängen, die dazu eingerichtet sind, mit außenseitigen Gewindegängen eines Wasserbehälters in Eingriff zu kommen, und
mit einer ringartigen Dichtfläche (112), die sich von einer innenseitigen Seitenwand des Verschlusses nach außen erstreckt, wobei die ringartige Dichtfläche einen im Wesentlichen horizontalen Kontaktbereich (114) aufweist, der dazu eingerichtet ist, eine wasserdichte Abdichtung gegen einen Hals eines Wasserbehälters bereitzustellen, wenn der Verschluss mit dem Wasserbehälter in Eingriff ist, **dadurch gekennzeichnet, dass** wenigstens die ringartige Dichtfläche (112), die innenseitige Deckfläche des Verschlusses, eine innenseitige Seitenwand des Verschlusses und innenseitige sowie außenseitige Oberflächen des Anschlusses aus einem zweiten Material sind, das weicher als andere, aus einem ersten Material hergestellte Teile des Verschlusses sind.

2. Wasserbehälterverschluss nach Anspruch 1, bei dem der Anschluss innerhalb der ringartigen Dichtfläche (112) angeordnet ist.

3. Wasserbehälterverschluss nach Anspruch 1, der weiterhin über einen Dichtring (116) verfügt, der sich von einer innenseitigen Deckfläche des Verschlusses nach außen erstreckt und eine wasserdichte Abdichtung zu einem Hals eines Wasserbehälters schafft, wenn der Verschluss mit dem Wasserbehälter in Eingriff ist.

4. Wasserbehälterverschluss nach Anspruch 3, bei dem der Dichtring (116) von einer im Wesentlichen sich verjüngenden Gestalt ist.

5. Wasserbehälterverschluss nach Anspruch 3, bei der eine innere Seitenwand (118) des Dichtrings im Wesentlichen vertikal ausgerichtet und eine äußere Seitenwand (120) des Dichtrings im Wesentlichen geneigt ist.

6. Wasserbehälterverschluss nach Anspruch 3, bei dem die äußere Seitenwand des Dichtrings und eine innenseitige Seitenwand des Verschlusses dazu eingerichtet sind, einen wasserdichten Verschluss eines Halses eines Wasserbehälters zu schaffen, wenn der Verschluss mit dem Wasserbehälter in Eingriff ist.

7. Wasserbehälterverschluss nach Anspruch 3, bei dem der Dichtring (116) näher an dem Anschluss als die ringartige Dichtfläche (112) angeordnet ist.

8. Wasserbehälterverschluss nach einem der Ansprüche 1 oder 3, der weiterhin eine Griffstruktur (110) aufweist, die an einer äußeren Oberfläche des Verschlusses angeordnet ist.

9. Wasserbehälterverschluss nach Anspruch 3, bei dem der Dichtring (116) aus dem zweiten Material ist.

10. Wasserbehälterverschluss nach einem der voranstehenden Ansprüche 1 bis 9, bei dem das zweite Material einstückig ist.

11. Wasserbehälterverschluss nach einem der voranstehenden Ansprüche 1 bis 10, bei dem das zweite Material durch Überspritzen auf dem ersten Material aufgebracht ist.

## Revendications

1. Un bouchon (20) de bouteille d'eau, comprenant :
au moins un orifice, s'étendant vers l'extérieur depuis une surface extérieure du bouchon, l'orifice s'étendant sur une longueur prédéterminée et étant configuré de façon à venir en engagement avec un organe de passage pour fournir un fluide vers un dispositif endoscopique ou recevoir un fluide depuis ce dispositif,
un filetage intérieur, configuré pour venir en engagement avec un filetage extérieur sur une bouteille d'eau, et
une plate-forme d'étanchéité annulaire (112), s'étendant vers l'extérieur à partir d'une paroi latérale intérieure du bouchon, la plate-forme d'étanchéité annulaire comprenant une zone de contact horizontale substantielle (114), configurée pour fournir une étanchéité à l'eau à un goulot d'une bouteille d'eau lorsque le bouchon est en engagement avec la bouteille d'eau, **caractérisé en ce qu'**au moins la plate-forme d'étanchéité annulaire (112), la surface supérieure intérieure du bouchon, une paroi latérale intérieure du bouchon et des surfaces intérieure et extérieure de l'orifice sont constitués d'un deuxième matériau plus souple que les autres parties du bouchon constituées d'un premier matériau.

2. Le bouchon de bouteille d'eau selon la revendication 1, dans lequel l'orifice est disposé à l'intérieur de la plate-forme d'étanchéité annulaire (112).

3. Le bouchon de bouteille d'eau selon la revendication 1, comprenant en outre un anneau d'étanchéité (116), s'étendant vers l'extérieur depuis une surface supérieure intérieure du bouchon et fournissant une étanchéité à un goulot d'une bouteille d'eau lorsque le bouchon est en engagement avec la bouteille d'eau.

4. Le bouchon de bouteille d'eau selon la revendication 3, dans lequel l'anneau d'étanchéité (116) est de forme sensiblement effilée.

5. Le bouchon de bouteille d'eau selon la revendication 3, dans lequel une paroi latérale intérieure (118) de l'anneau d'étanchéité est sensiblement verticale, et une paroi latérale extérieure (120) de l'anneau d'étanchéité est sensiblement inclinée.

6. Le bouchon de bouteille d'eau selon la revendication 3, dans lequel la paroi latérale extérieure de l'anneau d'étanchéité et une paroi latérale intérieure du bouchon sont configurées pour fournir une étanchéité à l'eau à un goulot d'une bouteille d'eau lorsque le bouchon est en engagement avec la bouteille d'eau.

7. Le bouchon de bouteille d'eau selon la revendication 3, dans lequel l'anneau d'étanchéité (116) est plus proche de l'orifice que la plate-forme d'étanchéité annulaire (112).

8. Le bouchon de bouteille d'eau selon l'une quelconque des revendications 1 ou 3, comprenant en outre une poignée (110), disposée sur une surface extérieure du bouchon.

9. Le bouchon de bouteille d'eau selon la revendication 3, dans lequel l'anneau d'étanchéité (116) est constitué du deuxième matériau.

10. Le bouchon de bouteille d'eau selon l'une quelconque des revendications précédentes 1 à 9, dans lequel le deuxième matériau est réalisé en une seule pièce.

11. Le bouchon de bouteille d'eau selon l'une quelconque des revendications précédentes 1 à 10, dans lequel le deuxième matériau est surmoulé sur le premier matériau.
